# EUROPEAN PATENT SPECIFICATION

(11) **EP 1 180 138 B1**
(45) Date of publication and mention of the grant of the patent: **04.07.2007**
(21) Application number: 00931437.8
(22) Date of filing: 25.05.2000
(51) Int. Cl.: C12N 9/22, C12N 9/10, C12Q 1/68, C12Q 1/25, C12Q 1/48

(54) **A POLYNUCLEOTIDE MOTOR, A MOTOR SYSTEM, THEIR PREPARATION AND USES**
EIN POLYNUCLEOTIDMOTOR, EIN MOTORSYSTEM, SEINE HERSTELLUNG UND VERWENDUNGEN
MOTEUR POLYNUCLEOTIDIQUE, SYSTEME DE MOTEUR, PREPARATION ET UTILISATIONS DE CES DERNIERS

(30) Priority: 25.05.1999 GB 9912179
(43) Date of publication of application: 20.02.2002
(73) Proprietor: University of Portsmouth, Portsmouth PO1 2UP (GB)
(72) Inventor: FIRMAN, Keith University of Portsmouth, Portsmouth PO1 2UP (GB)
(74) Representative: Watkins, David
(86) International application number: PCT/GB2000/002034
(87) International publication number: WO 2000/071681

(56) References cited:
- SCHAFER D A ET AL: "TRANSCRIPTION BY SINGLE MOLECULES OF RNA POLYMERASE OBSERVED BY LIGHT MICROSCOPY" NATURE (LONDON), vol. 352, no. 6334, 1991, pages 444-448, XP002149147 ISSN: 0028-0836
- YIN HONG ET AL: "Transcription against an applied force." SCIENCE (WASHINGTON D C), vol. 270, no. 5242, 1995, pages 1653-1657, XP002149148 ISSN: 0036-8075
- WANG MICHELLE D ET AL: "Force and velocity measured for single molecules of RNA polymerase." SCIENCE (WASHINGTON D C), vol. 283, no. 5390, 30 October 1998 (1998-10-30), pages 902-907, XP002149149 ISSN: 0036-8075
- JANSCAK PAVEL ET AL: "Analysis of the subunit assembly of the type IC restriction-modification enzyme EcoR124I." NUCLEIC ACIDS RESEARCH, vol. 26, no. 19, 1 October 1998 (1998-10-01), pages 4439-4445, XP002149123 ISSN: 0305-1048
- JANSCAK PAVEL ET AL: "DNA translocation blockage, a general mechanism of cleavage site selection by type I restriction enzymes." EMBO (EUROPEAN MOLECULAR BIOLOGY ORGANIZATION) JOURNAL, vol. 18, no. 9, 4 May 1999 (1999-05-04), pages 2638-2647, XP002149124 ISSN: 0261-4189
- MERNAGH DARREN R ET AL: "Protein-protein and protein-DNA interactions in the type I restriction endonuclease R.EcoR124I." BIOLOGICAL CHEMISTRY, vol. 379, no. 4-5, April 1998 (1998-04), pages 497-503, XP000946790 ISSN: 1431-6730
- ZINKEVICH VITALY ET AL: "The HsdR subunit of R cntdot EcoR124II: Cloning and over-expression of the gene and unexpected properties of the subunit." NUCLEIC ACIDS RESEARCH, vol. 25, no. 3, 1997, pages 503-510, XP002149125 ISSN: 0305-1048
- JANSCAK PAVEL ET AL: "The type I restriction endonuclease R.EcoR124I: Over-production and biochemical properties." JOURNAL OF MOLECULAR BIOLOGY, vol. 257, no. 5, 1996, pages 977-991, XP000946768 ISSN: 0022-2836
- SZCZELKUN MARK D ET AL: "Repercussions of DNA tracking by the type IC restriction endonuclease EcoR124I on linear, circular and catenated substrates." EMBO (EUROPEAN MOLECULAR BIOLOGY ORGANIZATION) JOURNAL, vol. 15, no. 22, 1996, pages 6335-6347, XP002149126 ISSN: 0261-4189
- PENNER MICHAL ET AL: "Phage T4-coded Stp: Double-edged effector of coupled DNA and tRNA-restiction systems." JOURNAL OF MOLECULAR BIOLOGY, vol. 249, no. 5, 1995, pages 857-868, XP000946769 ISSN: 0022-2836 cited in the application
- JANSCAK PAVEL ET AL: "Single amino acid substitutions in the HsdR subunit of the type IB restriction enzyme EcoAI uncouple the DNA translocation and DNA cleavage activities of the enzyme." NUCLEIC ACIDS RESEARCH, vol. 27, no. 13, 1 July 1999 (1999-07-01), pages 2638-2643, XP002149128 ISSN: 0305-1048
- PANNE DANIEL ET AL: "The McrBC endonuclease translocates DNA in a reaction dependent on GTP hydrolysis." JOURNAL OF MOLECULAR BIOLOGY, vol. 290, no. 1, 2 July 1999 (1999-07-02), pages 49-60, XP000946795 ISSN: 0022-2836

### SEQUENCE LISTING

<110> University of Portsmouth
<120> A polynucleotide motor, a motor system, their preparation and uses
<130> PCT/GB00/02034
<140> PCT/GB00/02034
   <141> 2000-05-25
<160> 6
<170> PatentIn Ver. 2.1
<210> 1
   <211> 37
   <212> DNA
   <213> Salmonella typhimurium
<400> 1
   cccccgtgca gaattcgagg tcgacggatc cgggggg 37
<210> 2
   <211> 14
   <212> DNA
   <213> Salmonella typhimurium
<220>
   <221> misc_feature
   <222> (14)
   <223> The end of sequence No. 2 is joined to the beginning of sequence No. 3 by a single biotin molecule.
<400> 2
   cagatgcacg tgag 14
<210> 3
   <211> 4
   <212> DNA
   <213> Salmonella typhimurium
<400> 3
   tcgc 4
<210> 4
   <211> 19
   <212> DNA
   <213> Salmonella typhimurium
<400> 4
   gccccgaaga acgttttcc 19
<210> 5
   <211> 39
   <212> DNA
   <213> Salmonella typhimurium
<220>
   <221> misc_feature
   <222> (39)
   <223> The end of Sequence No. 5 carries a biotin molecule.
<400> 5
   ctacggtacc gaaacgcgtg tcgggcccgc gaagcttgc 39
<210> 6
   <211> 43
   <212> DNA
   <213> Salmonella typhimurium
<400> 6
   catggatgcc atggctttgc gcacagcccg ggcgcttcga acg 43

## Description

The present invention relates to a nucleic acid sequence having bound thereto a particular complex involving a subunit of a restriction endonuclease, which complex is capable of translocating the polynucleotide without causing cleavage thereof; and its use, *inter alia,* in a molecular machine system.

Molecular machines have been described as molecules - on a nanometric scale - that have moving parts and do useful work. A molecular machine system may therefore be a multicomponent molecular machine. For such a machine or machine system to operate successfully, it must be based on a compact, stable molecular structure. Accordingly, theoretical studies of molecular machine systems have focused on inflexible, covalent structures, such as graphite- and diamond-like materials, working in a vacuum. However, it is unlikely that such theoretical systems can be built, in practice, in the near future.

On the other hand, the art of preparing polymeric structures is comparatively well-advanced. The drawback of these, however, is that they must fold appropriately in order to provide a usable structure. Protein folds, for example, are difficult to design in view of the lack of strong, natural complementarity of individual amino acids. Contrastingly, work has been carried out which shows that it is possible to design DNA-based structures, so that nucleic acids could be engineered to serve as scaffolds for complex molecular motor - and other - systems. The problem then is to provide a suitable motor or machine system that can appropriately interact with a DNA-based structure.

The study of molecular motors has mainly revolved around muscle proteins and similar macromolecular systems. However, biological motors also exist at the molecular level, and may provide suitable models for the developing nanotechnology industry. Of these, perhaps the most interesting from a biotechnological viewpoint (*eg* because of the potential use of the information content of DNA at the nanotechnological level) are those enzymes that manipulate nucleic acids. These include RNA polymerases; some enzymes involved in recombination (*eg* RecBCD); topoisomerases; and type I and III restriction enzymes. However, despite the potential of translocation, the mechanism by which DNA is moved through the protein complex is poorly understood. Furthermore, these enzyme systems are known not only to cause movement or tracking of the DNA, but also to have other effects, such as synthesis (in the case of polymerases); unwinding or breaking of DNA strands (such as by helicases); and cleavage (in the case of the restriction enzymes). Such effects clearly may render these systems undesirable or impossible to use as part of a molecular machine or machine system.

Nevertheless, the present invention surprisingly relates to a motor or machine system that is based on the movement or tracking of an enzyme, particularly a type I restriction enzyme, along DNA.

Type I restriction and modification (R-M) enzyme systems protect the bacterial cell against invasion of foreign DNA (such as viruses) by cleaving DNA which lacks a target specific N6-adenine methylation. The second physiological role of these systems is to restore full methylation of the target sites on the host DNA after DNA replication. Type I R-M enzymes (restriction endonucleases) are distinguished by the fact that the binding of an unmethylated recognition site elicits DNA cleavage at a distantly-located, nonspecific site on the DNA. ATP, which is required for DNA restriction, fuels translocation by the enzyme of the DNA from the recognition site to the site of cleavage.

Type I restriction endonucleases specifically recognise a non-palindromic DNA sequence (*eg* GAAnnnnnnRTCG for *Eco*R124I, where n is any base and R is a purine). Binding of the endonuclease to a non-modified recognition site activates a powerful ATPase activity, which fuels DNA translocation past the DNA-enzyme complex, while the enzyme remains bound to the recognition site. DNA is cleaved at positions where the DNA translocation stops - either due to a collision of two translocating enzyme molecules on two-site, linear DNA substrates, or due to the build-up of topological strain on circular molecules. The endonuclease does not turn over in the cleavage reaction; however, the ATPase activity continues for a long period of time after the cleavage is completed. DNA methylation activity of the type I R-M systems results in a transfer of a methyl group from a cofactor (*S*-adenosyl methionine or 'SAM') to the N-6 position of a specific adenine in each strand of the recognition sequence. Clearly, the cleavage that is associated with such translocation would be highly likely to negate the usefulness of such an enzyme as a potential motor.

Type I restriction-modification enzymes are composed of three different subunits (HsdR, HsdM and HsdS) encoded by the three *hsd* genes. All three subunits are absolutely required for restriction activity, while the HsdM and HsdS subunits are sufficient for modification activity and can also form an independent MTase. Type I R-M systems are grouped into four families, based on allelic complementation, protein homologies and biochemical properties of the enzymes. Type IA, IB and ID R-M systems are chromosomally encoded, while most type IC R-M systems are carried on large conjugative plasmids. The type IA family is typified by the *Eco*KI and *Eco*BI enzymes, type IB by *E*coAI and type IC by *Eco*R124I. *Eco*KI forms a stable R₂M₂S₁ complex; however, the independent EcoKI MTase (M₂S₁) is a relatively weak complex, dissociating into an inactive M₁S₁ species and free HsdM subunit. The purified *Eco*BI restriction endonuclease exists in a number of different stoichiometric forms including R₂M₂S₁, R₁M₂S₁ and R₁M₁S₁. The type IB restriction endonuclease *Eco*AI is a weak complex that dissociates into MTase and HsdR subunit when purified.

It has already been shown (Janscak in Nucleic Acids Research 26 (19) 4439-4445 (1998) that the purified *Eco*R124I restriction endonuclease is a mixture of two species, which have a subunit stoichiometry of R₂M₂S₁ and R₁M₂S₁, respectively. Only the former species was found to have endonuclease activity. However, the R₂M₂S₁ complex is relatively weak, dissociating into free HsdR subunit and the restriction-deficient R₁M₂S₁ assembly intermediate, which appears to be a very tight complex. Although the R₁M₂S₁ complex cannot cleave DNA, it is capable of nicking one strand of the DNA. However, up until the present invention, there had been no indication that the R₁M₂S₁ complex is itself capable of translocating the DNA in spite of the fact that it does not cause cleavage thereof.

For one thing, no satisfactory method had yet been found for producing the restriction-deficient R₁M₂S₁ complex ("the R₁ complex") preferentially over the R₂M₂S₁ endonuclease, to enable - in practice - synthesis of an R₁M₂S₁ enzyme-polynucleotide complex on a useful scale. We have now found, as described further below in Example 1, that the synthetic Stp-like polypeptide, Stp₂₋₂₆, shifts the equilibrium between the HsdR₂M₂S₁ and HsdR₁M₂S₁ subunit complexes towards the latter form. Stp polypeptide is the anti-restriction determinant of bacteriophage T4 having 26 amino acids, whose presence results primarily in the R₁M₂S₁ restriction-deficient complex.

In addition, we have produced a hybrid HsdR subunit that has the same amino acid sequence as that predicted for the HsdR subunit of *Eco*prrI. Studies with a hybrid endonuclease comprising the MTase from *Eco*R124I and the HsdR(prrI) subunit have shown that this hybrid enzyme can only cleave DNA in the presence of extremely high concentrations of HsdR(prrI), which indicates that this assembly has an even weaker R₂-complex than that of *Eco*R124I and would also be suitable for R₁-complex production. Furthermore, a point mutation of *Eco*AI has been shown to translocate without cleavage (Janscak et al in Nucleic Acids Research 27(13), 2638-2643) (1999)); single amino acid substitutions in the HsdR subunit of the type IB restriction enzyme EcoAI uncouple the DNA translocation and DNA cleavage activities of the enzyme and could also be a motor of this type.

With the ability preferentially to produce the R₁ complex, the production of a polynucleotide, such as DNA, having complexed therewith an enzyme, such as one comprising the R₁ complex, has been achieved and has surprisingly been found to be capable of translocating the polynucleotide, in spite of the fact that it is not able to cause cleavage thereof.

We have therefore now identified a complex between a polynucleotide sequence, such as a DNA sequence, and an enzyme, such as R₁M₂S₁, capable of translocating the nucleic acid sequence without causing cleavage thereof or other apparent effects that would detract from its usefulness, such as polymerase activity. Furthermore, we have also found that such a (translocation but non-restriction) enzyme-polynucleotide complex can provide the motor for use in the machinery according to the present invention, which motor may be powered, for example, by the presence of ATP and magnesium ions (Mg⁺⁺).

In a first aspect, the present invention provides a molecular motor system comprising a nucleic acid sequence having bound thereto:
(1) at a first, proximal, region of the nucleic acid, an enzyme capable of translocating the nucleic acid sequence, said enzyme remaining bound to said proximal region of the nucleic acid, as a complex therewith, during the translocation; and
(2) at a second, distal, region of the nucleic acid, a bound substance capable of remaining bound to the nucleic acid sequence at said distal region during the translocation;
characterised in that the enzyme comprises a type I restriction-modification enzyme having HsdR, HsdS and HsdM sub-units exhibiting the stoichiometric form HsdR₁M₂S₁.

The specification also discloses an enzyme capable of binding to a nucleic acid sequence, which enzyme is not capable of restriction of the sequence, characterised in that the enzyme is capable of translocating with respect to the sequence. Preferably, the enzyme is also capable of nicking the sequence; that is, in the case of a DNA sequence, of breaking one of the double strands of the DNA sequence without breaking the other strand thereof.

Preferably, the nucleic acid sequence is a polynucleotide, such as DNA. The DNA may comprise linear or circular DNA; more preferably, linear DNA.

Preferably, the enzyme is derived from a restriction enzyme, such as one derived from the HsdR subunit, such as one derived from a type I restriction-modification enzyme; more preferably it is derived from a type IC R-M enzyme, such as *Eco*R124I or EcoprrI. The importance of HsdR as the subunit responsible for DNA cleavage and ATP-binding suggests other approaches that should produce a restriction enzyme, which can translocate DNA without cleavage. One method would be to produce a mutation within the *hsdR* gene that inactivates the DNA cleavage event without losing the ATPase activity. A restriction enzyme comprising such a mutant HsdR subunit would also be a molecular motor, equivalent in many respects to the R₁-complex; although it will produce bi-directional translocation. A short motif of amino acids, common to many endonucleases, would be the most likely site for such mutations. Other mutations may exist that alter the subunit assembly of the R₂-complex and stabilise the R₁-complex in a manner similar to that observed with HsdR(prr); such mutants would therefore also produce a useful molecular motor. Especially preferred is when the enzyme is derived from a type I endonuclease and exhibits the stoichiometric form R₁M₂S₁, especially the R₁M₂S₁ derived from *Eco*R124I.

Accordingly, the present invention further provides the use of the R₁-complex for the preparation of a polynucleotide motor in which a nucleic acid sequence (eg a polynucleotide (eg DNA)) to which it is bound or complexed is translocated but not cleaved. Hence, a particularly preferred aspect of the present invention comprises an R₁M₂S₁-DNA complex having bound thereto a substance capable of remaining bound to the DNA during translocation of the DNA, whereby the bound substance is itself translocated during translocation of the DNA.

Hereinafter, the nucleic acid sequence having the (translocating but not restricting) enzyme bound thereto may be referred to as "the polynucleotide-enzyme complex". In this context, references to a polynucleotide may include references to other nucleic acid sequences, unless specifically stated to the contrary.

Therefore, with the polynucleotide-enzyme complex able to translocate the bound substance, there is further provided a method for translocating a substance bound to a nucleic acid sequence from a distal region of the nucleic acid sequence towards a proximal region, which method comprises:
(a) (i) providing at the distal region of the nucleic acid sequence a bound substance, or
   (ii) binding a substance to the distal region of the nucleic acid sequence; and
(b) (i) providing at the proximal region a complex of the nucleic acid sequence with an enzyme, or
   (ii) complexing an enzyme to the proximal region of the nucleic acid sequence, and
(c) activating the enzyme, whereby the enzyme translocates the nucleic acid sequence, including the bound substance, from the distal region towards the proximal region;
characterised in that the enzyme comprises a type I restriction-modification enzyme having HsdR, HsdS and HsdM sub-units exhibiting the stoichiometric form HsdR₁M₂S₁.

Activation of the enzyme will depend upon the particular enzyme chosen and, in the case of the R₁M₂S₁ complex, will conveniently comprise the presence of ATP, as demonstrated in Example 2 below, where the ability of an R₁M₂S₁-DNA complex to translocate, in the presence of ATP, a bound substance comprising a XhoI restriction site linked to a chemiluminescent enzyme is shown. In Example 2, the ATP is present with Mg⁺⁺ in a restriction or cleavage buffer of particular composition. However, the person skilled in the art will understand that a range of buffers or buffer conditions, determinable by routine trial and error, will be suitable for activating the enzyme according to step (c) of the method of this invention. Preferred buffers, though, include freshly-prepared dithiothreitol, and ATP is added at a concentration of preferably greater than 0.5mM, with about 2mM being sufficient to result in full enzyme activity.

The bound substance will depend upon the particular use to which the polynucleotide motor system according to the present invention is to be put, which is described further below. The bound substance may itself comprise one or more components or ligands; therefore, the bound substance may comprise:
(a) a substance which is required to be translocated; or
(b) means for binding the substance (which is required to be translocated) to the polynucleotide-enzyme complex; or
(c) both (a) and (b) together.

For example, the bound substance may initially comprise a binding ligand that can bind to a substance in solution, such as a test compound or other material required to become attached to the polynucleotide-enzyme complex (such as chemiluminescent enzymes, magnetic beads or carbon-based 'gears') which, once attached, together with the ligand themselves, form the bound substance; or the bound substance may comprise a specific DNA sequence to which DNA-binding protein(s) may bind. Alternatively, the bound substance may not involve a binding ligand, but may be directly bound to the polynucleotide, such as fluorophors or the like. The bound substance therefore may interact with the environment external to the polynucleotide-enzyme complex to produce a detectable and/or measurable effect, such as a chemical, biological or physical reaction, eg the emission of light, electric current or movement.

Many of the uses to which the polynucleotide motor system of the present invention can be put require, in practice, the polynucleotide-translocating enzyme complex to be anchored, such as bound to a solid surface, rather than freely mobile in a solution. Accordingly, the present invention further provides a nucleic acid sequence having bound thereto an enzyme capable of translocating the nucleic acid sequence without causing cleavage thereof (the "polynucleotide-enzyme complex"), which polynucleotide-enzyme complex is attached to a solid support.

Preferably, the nucleic acid sequence also has bound thereto a substance (the 'bound substance') capable of remaining bound to the nucleic acid sequence during translocation, whereby the bound substance is itself translocated, relative to the region of binding of the enzyme to the nucleic acid sequence, during translocation. More preferably, the polynucleotide-enzyme complex is substantially linear, prior to translocation; and especially preferred is when only one end thereof is attached to the support, enabling its free end to be available for binding thereto of the bound substance and thus capable of motion relative to the support.

One way of anchoring the polynucleotide-enzyme complex is to bind it to a binding ligand that is itself capable of binding to a substrate coated on the solid support. Hence, the means of attachment between the polynucleotide-enzyme complex and the solid support may be direct or indirect. An example of indirect attachment is wherein the binding ligand is biotin and the substrate is a biotin-binding protein such as avidin, streptavidin or Neutravidin (available from Pierce & Warriner (UK) Ltd, Upper Northgate Street, Chester, UK), any of which may conveniently be coated on a solid support, as described by Bayer et al in A Sensitive Enzyme Assay for Biotin, Avidin, and Streptavidin in Analytical Biochemistry 154 (1), 367-70 (1986). For example, plasmid DNA can be copied (using PCR) to yield a linear DNA fragment, having biotin attached to its end near the recognition site for the *Eco*R124I enzyme and the DNA can then be attached, via the biotin, to a streptavidin-coated support, followed by assembly of the R₁ complex at the recognition site on the DNA, as described in Example 3.

Conveniently, the solid support comprises a component, such as a chip of a surface plasmon resonance (SPR) machine, which can be used to monitor not only the binding of the polynucleotide-enzyme complex to the support but also reactions of the complex and use of the motor system.

Alternatively, the support or binding ligand may comprise a microparticle containing a scintillant, such as those used in conventional proximity assays.

Accordingly, the present invention further provides a molecular motor system comprising a nucleic acid sequence having bound thereto:
(1) an enzyme capable of translocating the nucleic acid sequence, said enzyme remaining bound to a proximal region of the nucleic acid, as a complex therewith, during the translocation, and
(2) a solid support;
characterised in that the enzyme comprises a type I restriction-modification enzyme having HsdR, HsdS and HsdM sub-units exhibiting the stoichiometric form HsdR₁M₂S₁.

Optionally, a substance capable of remaining bound to the nucleic acid sequence during translocation may be bound thereto. During translocation, the bound substance is itself translocated, relative to the region of binding of the enzyme to the nucleic acid sequence.

A binding ligand, such as biotin, may be bound to the nucleic acid sequence. A substrate may be provided, such as avidin or streptavidin, for binding the binding ligand thereto and for anchoring the nucleic acid sequence thereto when the nucleic acid sequence is in solution, such as by being coated on the surface of the solid support.

Preferably, the substrate is itself or is associated with solid means for monitoring activity of the polynucleotide-enzyme complex, such as a streptavidin-coated chip of an SPR apparatus.

Depending upon the use to which the polynucleotide motor of the present invention is to be put, the bound substance may itself make use of a binding ligand/substrate (eg biotin/streptavidin or avidin) interaction. For example, the bound substance may itself comprise biotin, or streptavidin or avidin. Commercially-available substances suitable for binding to the polynucleotide-enzyme complex include streptavidin- or biotin-coated magnetic beads, or chemiluminescent enzymes bound to streptavidin or biotin. Clearly, for many uses of the polynucleotide-enzyme complex of the present invention, the biotin and streptavidin or avidin may be interchanged, and the description and examples herein should be interpreted accordingly. Other substances suitable for binding to the polynucleotide-enzyme complex and making use of the biotin-streptavidin or -avidin, or other binding ligand-substrate interactions are available to those skilled in the art, such as DNA-binding proteins or enzymes, which bind specific sequences in the DNA.

A particular example of the use by a polynucleotide motor system according to the present invention of such a bound substance/streptavidin interaction is as a so-called 'molecular pulley' or 'molecular fish-hook and line'. Using the streptavidin-coated SPR chip-immobilised polynucleotide motor system described in Example 2, to the free end of which an oligonucleotide-attached biotin has been ligated, a streptavidin-attached chemiluminescent molecule can be 'fished' out of solution by the biotin and, in the presence of ATP to fuel translocation, pulled towards the streptavidin-coated chip, where its presence can be monitored by detection of the emission of light, as described in Example 3. This model of a molecular pulley demonstrates the potential use of the polynucleotide motor-pulley system according to the present invention in pulling molecules, such as a test substance or ligand, out of solution towards a solid surface, where they can be detected and/or isolated and/or their activity measured and/or otherwise tested.

Accordingly, the present invention provides a method for capturing a test substance in solution, suspension or dispersion and bringing it into association with a solid surface, which method comprises:
(a) providing a molecular motor system comprising a nucleic acid having:
   (i) a proximal region of the nucleic acid to which is bound an enzyme capable of translocating the nucleic acid sequence, said enzyme remaining bound to said proximal region of the nucleic acid, as a complex therewith, during the translocation;
   (ii) a distal region of the nucleic acid which enables the test substance to be captured, the distal region and/or the test substance being adapted for that purpose, and
   (iii) a solid surface attached to the nucleic acid;
(b) bringing the distal region of the polynucleotide-enzyme complex into contact with the test substance, whereby the test substance is captured, and
(c) activating the enzyme, whereby the enzyme translocates the polynucleotide, including the test substance, from the distal region towards the solid surface;
characterised in that the enzyme comprises a type I restriction-modification enzyme having HsdR, HsdS and HsdM sub-units exhibiting the stoichiometric form HsdR₁M₂S₁.

This method has particular application in the pharmaceutical industry, where current screening methods used for testing, eg. protein-drug interactions, rely on two-component systems. In many such systems, interaction between the two components is detected by a mechanism involving light emission produced by a radiolabelled drug or protein interacting with a scintillant, or fluorescence resulting from proximal interaction of the two-component fluorescent chemicals (quantum exchange between these two chemicals results in detectable light emission). Such systems involve, for example, a drug being anchored to the scintillant, which may exist as a bead, or to one of the fluorescent chemicals, and a complex being identified by light emission when a known control protein, which is either radioactive or carries the other fluorescent chemical, binds to the drug. Proteins or other test compounds to be screened for their ability to bind the drug are tested for their ability to displace the control protein bound to the drug/bead complex.

However, these detection systems are difficult to set up and it is difficult to isolate the resulting two-component complexes. Also, although the detection of these complexes appears simple, in practice, this has also been found to be less than reliable. In addition, the use of radioactive samples is a problem for reasons of both safety and disposal.

On the other hand, the molecular motor-based 'fishing hook' according to the present invention will not only allow relatively easy detection of interaction between many drugs and many proteins by employing an array method with direct detection of drug-protein interaction using a proximity assay, but will also allow isolation of the resulting complex. Attachment of the drug to the end of the DNA can be achieved through ligation of suitable oligoduplex synthesised with bound drug (or through a biotin-streptavidin-drug complex). Furthermore, this system is designed to work at the molecular level and is therefore capable of detecting single molecules interacting with each other.

This technology is a version of Scintillation Proximity Assays (SPAs). The motor would be attached to a bead (see above) and the proteins to be screened for interaction with drugs would be radiolabelled. Since the DNA translocated by the motor could be produced with a wide variety of attached drugs, this system would allow very large-scale screening of expression libraries against large numbers of drugs. In addition, because there is both a time delay and physical movement of the drug-enzyme complex not only will the system allow direct detection of such interactions, but it would also only detect strong binding complexes (as others are more likely to dissociate before detection). This system should greatly advance the current miniaturisation of SPA and lead to use of such assays on a nanometric scale.

Accordingly, the present invention further provides a method for screening a test substance for a predetermined biological, chemical or physical activity, which method comprises:
(a) providing a solution, suspension or dispersion of the test substance, either (i) itself or (ii) in association with a first interactive substance, capable of providing or inducing a detectable reaction in a second interactive substance;
(b) providing a polynucleotide motor system comprising a nucleic acid having bound thereto:
   (1) at a first, proximal region of the nucleic acid, an enzyme capable of translocating the nucleic acid sequence, said enzyme remaining bound to said proximal region of the nucleic acid, as a complex therewith, during the translocation, and
   (2) at a second, distal region of the nucleic acid, a bound substance capable of remaining bound to the nucleic acid sequence at said distal region during the translocation;
      wherein the nucleic acid is attached to a solid support and wherein:
      (i) the bound substance is further capable of binding to a test substance exhibiting the predetermined activity, and
      (ii) the bound substance is itself or the solid support comprises the second interactive substance;
(c) activating the polynucleotide motor system to effect translocation, and
(d) monitoring the presence or absence of the detectable reaction during or after translocation;
characterised in that the enzyme comprises a type I restriction-modification enzyme having HsdR, HsdS and HsdM sub-units exhibiting the stoichiometric form HsdR₁M₂S₁.

The present invention therefore further provides a substance (such as a chemical compound) for use in industry, medicine or agriculture, whenever identified or which is capable of being identified by the screening method of this invention.

According to this screening method, for example in the presence of ATP, the polynucleotide motor system of the present invention will result in translocation of the polynucleotide, together with the bound substance to which may be bound a test substance exhibiting the predetermined activity. Translocation of the bound substance-test substance complex will result in the test substance, optionally together with its associated first interactive substance, being pulled towards the solid support and hence the second interactive substance, resulting in the detectable reaction. Alternatively, the bound substance-test substance complex will itself result in the detectable reaction, which will be detected as this complex approaches the solid support.

Examples of detectable and, preferably also, measurable reactions include chemiluminescence, as mentioned with respect to the model system above; magnetism; electric current; chemical reaction; radioactivity; scintillation; or the like.

Accordingly, further uses for the polynucleotide motor according to this invention include its use as an intelligent switch, such as by the use of a fluorescent marker on DNA, which is activated by quantum exchange following movement of the DNA carrying a first fluorophore towards a second fluorophore. The photon emitted by the quantum exchange(s) can be detected and used to operate other equipment. Hence, activation of the equipment can be governed by the DNA sequence, thereby making the 'switch' 'intelligent'. This has applicability to DNA sequence detection on a nanotechnological scale or for programmed control of instrumentation.

Another use arises from the fact that translocation of DNA with an attached magnetic bead would rotate the bead in a spiral motion around the DNA strand due to the helical nature of DNA. Such motion of a magnet around a conductor would produce an electric current. Since there is evidence that DNA can conduct electricity; such a system could provide a molecular dynamo, and the generated electricity could be tapped to switch transistors.

The invention further provides for use of:
a type I restriction-modification enzyme having HsdR, HsdM and HsdS sub-units in the stoichiometric form HsdR₁M₂S₁ in a molecular motor system as defined in any one of claims 1 to 9.

The present invention will now be illustrated by the following non-limiting examples.

### Example 1 - Preparation of R₁-complex Molecular Motor

### Example 1A - Preparation of Plasmid pCFD30

Plasmid pCFD30 is a recombinant plasmid produced by inserting an oligoduplex of formula
CCGTGCA**GAATTCGAGGTCG**ACGGATCCGG
GGCACGT**CTTAAGCTCCAGC**TGCCTAGGCC
containing a single recognition site (identified in **bold typeface)** for *Eco*R124I (the molecular motor) (Taylor et al., Substrate Recognition and Selectivity in the Type IC DNA Modification Methylase M.EcoR124I in Nucleic Acids Research 21 (21) (1993)) into the unique *Sma*I site of pTZ19R (Mead et al., Single-Stranded DNA 'Blue' T7 Promoter Plasmids: A Versatile Tandem Promoter System for Cloning and Protein Engineering in Protein Engineering 1 67-74 (1986))using standard methods described by Maniatis et al in Molecular Cloning: A Laboratory Manual, Cold Harbor Laboratory, New York (1982). The DNA sequence at the *Sma*I site (below, identified in *italics*) of pCFD30 was found to be CCCCCGTGCA**GAATTCGAGGTCG**ACGGATCCGGGGGG, which shows the orientation of the *Eco*R124I recognition sequence in the plasmid.

### Example 1B - Preferential Production of the R₁-complex/Molecular Motor

10nM pCFD30 plasmid DNA, prepared as described above, was incubated at 37°C in 25µl of cleavage buffer (50mM Tris-HCl pH8.0; 1mM dithiothreitol (DTT); 10mM MgCl₂; 50mM NaCl). To this was added 50nM MTase(R124) and 40nM HsdR(R124) - these concentrations ensure primarily R₁-complex formation.

To ensure R₁-complex formation, the synthetic Stp-like polypeptide (described by Penner et al in Phage T4-coded Stp: Double-edged Effector of Coupled DNA- and tRNA-Restriction Systems in Journal of Molecular Biology 249 (5), 857-68 (1995)), Stp₂₋₂₆ was used to promote dissociation of any R₂-complex. Accordingly, the same reactions as above were carried out in the presence of 100nM Stp₂₋₂₆ polypeptide.

Alternatively, the above procedure can be followed, but using 40nM HsdR(prrI) in place of HsdR(R124).

### Example 1C - Confirmation of production of the R₁-complex

To confirm production of the R₁-complex and to ensure no R₂-complex is present, a cleavage test is performed. If only R₁-complex is present there should be no cleavage product.

2mM ATP was added to the product of Example 1B to initiate the reaction, and the samples were incubated for 30 minutes. A cleavage-positive control was also performed in which an excess (250nM) of HsdR (R124) was added in order to promote formation of R₂-complex. 10µl samples were run on 1% agarose gels.

The R₂-complex was found to cleave the plasmid DNA, as expected. No cleavage was observed for the R₁-complex, confirming production of R₁-complex. Either mixture can be used for all subsequent motor experiments.

### Example 2 - Translocation of DNA by an R₁-complex

This example provides the first confirmation of DNA translocation by the R₁-complex: circular plasmid DNA carrying a chemiluminescent enzyme was translocated and the translocation shown by cleavage with *Xho*I. The model used depends upon the fact that a unique restriction enzyme cleavage site (for *Xho*I) can be 'buried' in the translocation complex preventing cleavage by *Xho*I*.* In this example, the cleavage of circular DNA carrying a biotin molecule to which a streptavidin-linked chemiluminescent enzyme can be attached was investigated. The presence of the chemiluminescent enzyme should halt translocation by collision with the translocating R₁-complex and 'bury' the *Xho*I site within the translocation complex. When the *Xho*I site is buried in the translocation complex (translocation will be stopped by the presence of the chemiluminscent enzyme), there is no linearisation of the plasmid by *Xho*I. This event is independent of the direction of translocation.

### Example 2A Preparation of DNA-bound Chemiluminescent Enzyme

Plasmid pCFD30 (as defined in Example 1A) was linearised with *Xmn*I and ligated to an excess of oligoduplex (CAGATG**CACGTG**AG*TCGC) containing a *Xho*I site (identified by **bold typeface**) and a single biotin molecule (obtained from Cruachem Ltd, Glasgow) linked to thymine (*T) to produce pCFD30-biotin. Recombinants were identified by *Xho*I cleavage. The presence of a single copy of the oligoduplex was produced by *Xho*I cleavage followed by re-ligation and confirmed by DNA sequence analysis of the resulting recombinants.

An excess of streptavidin-linked chemiluminescent enzyme (streptavidin-bound horseradish peroxidase (S-HRP), available from Pierce & Warriner (UK) Ltd, Upper Northgate Street, Chester, UK) was added to the plasmid, and the complex was purified from surplus enzyme using ethanol precipitation (Maniatis, *ibid*, Example 1A).

The presence of the DNA-bound enzyme was confirmed by a simple chemiluminescent measurement. 100ng of the pCFD30-biotin/S-HRP plasmid were spotted onto a nylon membrane soaked in chemiluminescent substrate (SuperSignal (Registered Trademark) Substrate from Pierce & Warriner (UK) Ltd, Upper Northgate Street, Chester, UK). The membrane was incubated at 37°C to activate the enzyme and the spot visualised using X-ray film. Controls of pCFD30 and pCFD30-biotin were spotted onto the same membrane.

A positive light emission was obtained for the pCFD30-biotin/S-HRP plasmid only, indicating a DNA-bound chemiluminescent enzyme.

### Example 2B - Confirmation of Translocation by R₁-complex

An R₁-complex was produced and confirmed as not cleaving pCFD30 as described in Example 1. This R₁-complex was incubated with an equimolar concentration of pCFD30-biotin with bound chemiluminescent enzyme prepared as above. ATP was added, using the method described in Example 1. After 15 min at 37°C, the plasmid was subjected to cleavage with 10 units of *Xho*I*.* In addition, pCFD30, pCFD30-biotin, pCFD30-biotin/S-HRP and pCFD30-biotin/S-HPP with R₁-complex, but without ATP were also subjected to *Xho*I cleavage.

All plasmids except the pCFD30-biotin/S-HRP with ATP were cleaved by the *Xho*I, producing linear DNA (detected following gel electrophoresis). The addition of γ-S-ATP (a non-hydrolysable analogue of ATP, incapable of supporting translocation) instead of ATP also produced linear DNA. Hence, the *Xho*I site was 'buried' in the stalled translocation complex produced by the action of ATP, preventing cleavage. Translocation in either direction accomplishes this process.

Therefore, the R₁-complex is capable of ATP-driven translocation and can be used as a molecular motor. This is the first confirmation of translocation (as opposed to ATPase activity) by the R₁-complex.

### Example 3 - Surface-Attached Molecular Motor

Plasmid pCFD30 DNA was copied using PCR (using Universal primer with biotin attached at the 5'-end (available from Cruachem Ltd., Todd Campus, West of Scotland Science Park, Acre Road, Glasgow G20 0UA) and a primer overlapping the unique *Xmn*I site: GCCCCGAAGAACGTTTTCC) to yield a linear DNA fragment with biotin attached at one specific end (near the recognition site for the R₁ enzyme). The PCR product was attached to the streptavidin-coated chip of an SPR (surface plasmon resonance) machine (Biacore X available from Biacore AB, Meadway Technology Park, Stevenage, Herts., UK). Attachment was monitored using SPR to confirm that no more PCR product could bind to the chip. Biotin was attached to another oligoduplex (as in Example 2A), which was ligated to the other end of the chip-bound PCR product; again, attachment was monitored using the SPR.

This additional oligoduplex had a restriction enzyme target site near one end, accessible for cleavage by the restriction enzyme (*Xho*I).

The R₁ complex, prepared according to Example 1 (the molecular motor), was attached to the PCR product (the target site was near the chip) and attachment monitored by SPR. Addition of ATP resulted in translocation of the DNA. Following translocation, the *Xho*I target site is inaccessible because it is buried in the translocation complex, as seen in Example 2.

To monitor the process of translocation and to confirm the 'fishing hook' model, a streptavidin-bound enzyme capable of chemiluminescence (S-HRP, as described in Example 2), was 'fished' out of solution by the biotin bound at the end of the DNA molecule. The presence of the enzyme was monitored by light emission from the chip. Cleavage by *Xho*I releases back to solution all non-translocated chemiluminescent enzyme. Chemiluminescent enzyme present in solution was removed by repeated washing.

### Example 4 - Further Experiments on Surface Attachment of the Motor

The experiments described below were performed using a streptavidin-coated chip (Biacore SA5 available from Biacore AB, Meadway Technology Park, Stevenage, Herts., UK) from a surface plasmon resonance machine (BIAlite 2000), as this machine allows the real-time monitoring of attachment of molecules to the surface of the chip. It also allows the real-time monitoring of release of molecules from the chip surface.

To further investigate the function of the motor when attached to a solid surface via one end of a DNA molecule, the following experiments were carried out:

### Example 4A - Preliminary Surface-Attachment of DNA

An oligonucleotide (CTACGGTACCGAAACGCGTGTCGGGCCCGCGAAGCTTGCₓ) carrying a biotin molecule (ₓ) at one end was synthesised by Cruachem. Attachment of the oligo to the surface was monitored by SPR. This oligo was annealed to a complementary oligo (CATGGATGCCATGGCTTTGCGCACAGCCCGGGCGCTTCGAACG) to give an oligoduplex with a biotin attached and with a suitable 6-base-pair "sticky-end" at the 3'end (biotin end) to allow ligation of another DNA molecule. Annealing of this second oligo was also monitored by SPR. The running buffer (buffer 1) used was 10mM Tris-HCl (pH8), 10mM MgCl₂, 100mM NaCl, 1mM DTT

### Example 4B - Determination of Optimum Motor Density

Several experiments were performed to determine the maximum density at which such an oligoduplex could be bound to a streptavidin-coated chip of a surface plasmon resonance machine. This was accomplished by repeated passage of dilute (<1nM, but known concentration) solutions of oligonucleotide over the chip. When no further binding was observed this total concentration was the capacity of the chip. It was confirmed that, at low densities, the remaining streptavidin sites on the chip could be blocked using free biotin and that no more oligoduplex could then be bound to the chip.

However, there was found to be considerable variation in the capacity of each batch of chips presumably due to small variations in the surface area. Therefore, the number of molecules present on each chip tested was calculated. Using these data, it was possible to determine which chips carried approximately one molecule every about 100nm² on the chip. This was found to be the best density for the experiment described below; at lower densities, any changes in the SPR data were difficult to measure, whereas higher densities gave more random results suggesting interference between adjacent motors/DNA molecules.

### Example 4C - Experiments using Linear DNA

Plasmid pCFD30 DNA was copied using PCR (using Universal primer available from Cruachem Ltd., Todd Campus, West of Scotland Science Park, Acre Rod, Glasgow G20 0UA) and a primer overlapping the unique *Xmn*I site: GCCCCGAAGAACGTTTTCC) to yield a linear DNA fragment. This linear DNA was cleaved with *Kpn*I to produce a suitable complementary "sticky-end". The PCR product was attached to the streptavidin-coated chip of an SPR by ligation to the oligoduplexes attached to the surface of the chip using standard procedures described in Maniatis (*ibid*, Example 1). The chip was washed free of ligase and any unligated linear DNA was removed using 1% SDS in the buffer detailed above (this can also be used to remove *Eco*R124I enzyme from the DNA on the chip). The data from the SPR showed ligation was successful.

Purified *Eco*R124I in the form of the R₁-complex (100nM MTase + 100nM HsdR (R124 with and without 100nM Stp, or prr[which does not require Stp]), or the R₂-complex (100nM MTase + 500nM HsdR) in cleavage buffer (Example 1B), was allowed to bind to the DNA (monitored by SPR). Upon addition of 2mM ATP a large release of material from the chip was observed for both complexes. Confirmation that the motor was presented as an R₁-complex was determined by the method described in Example 1B.

Interestingly, over any time period, there was a greater release of material for the R₂-complex than the R₁-complex and the rate of release was also greater for the R₂-complex. Washing of the chip with buffer 1 (Example 4A), after the translocation assays, followed by further addition of ATP showed that the R₁-complex was capable of further changes but the R₂-complex was not.

This is due to translocation, by the motor, of the DNA followed by displacement of the biotin-streptavidin linkage to the surface, thereby releasing the motor. The R₂₋complex is capable of bi-directional translocation and can displace all bound motors, while the R₁-complex is uni-directional and only some (-50%) of the motors are displaced from the surface. Addition of further ATP to the R₁-complex allows further translocation and thereby further displacement of the motor. Therefore, the R₁-complex is also capable of re-setting after translocation.

### Example 4 - Conclusion

The translocation of DNA by the motor leads to displacement of the DNA from the surface of the chip. Therefore, both R₁-complex and R₂-complex are capable of translocation. The data from the R₁-complex also shows that not all the motors are displaced suggesting that some motors are translocating away from the surface without displacement.

### Example 5 - Investigation of the Disruption by Translocation of the Biotin-Streptavidin Linkage

Example 2 hereinabove confirmed translocation by the R₁-complex. However, although the concept of the biotin-streptavidin-HRP complex 'blocking' translocation appears to be the most logical explanation of these results, another explanation could be that translocation is simply blocked by the lack of available DNA for translocation and by chance the *Xho*I site becomes 'buried' in the resulting stalled complex. Therefore, it was necessary to investigate any displacement of the biotin-streptavidin linkage.

The following experiment was undertaken to show that displacement of the motor (R₁-) from the surface of the SPR chip was not just the result of the collision of the motor with the surface but a result of a displacement of the biotin-streptavidin bond by translocation.

### Example 5A - Use of Linear DNA

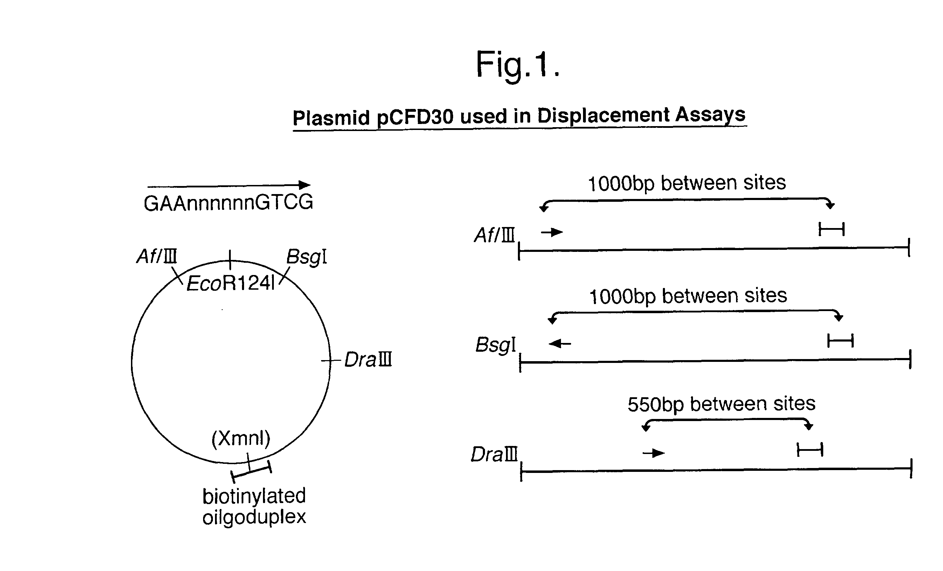

The pCFD30-biotin plasmid, produced as described above, was used to produce three linear plasmids by cleavage with AflIII, BsgI or DraIII, respectively, (shown in Figure 1). Cleavage of 200ng of pCFD30-biotin with the respective restriction enzymes (following manufacturer's instructions - New England BioLabs) produced the required plasmids, each having the biotin molecule at different distances (and orientations) from the s_{R124} site. The oligoduplex contains a unique *Xho*I site, which should be inaccessible if 'buried' under the translocating motor/biotin-streptavidin-HRP (as previously described). However, if the biotin-streptavidin bond is broken by the translocating motor, then addition of excess streptavidin, after addition of ATP, should prevent HRP from rebinding to the biotin on the DNA.

Streptavidin-HRP (Pierce & Warriner Cat No. 21126) was added to the plasmid (excess over the plasmid concentration) and excess removed by ethanol precipitation of the DNA using standard techniques (Maniatis *ibid,* Example 1). The presence of the linked HRP was assayed on a sample (5-10ng) of DNA using the Pierce Chemiluminescence substrate Supersignal (Cat no. 34080) as described by the manufacturer following "spotting" of diluted samples onto nylon membrane.

The HRP-linked plasmid (100nM) was incubated with equimolar concentration of MTase + HsdR (R124 or prr) (R₁-complex) motor, as described in Example 2B. Translocation was commenced by addition of 2mM ATP.

For the blockage of translocation assay (as described in Example 2 for circular DNA), cleavage of the plasmid was assayed using *Xho*I enzyme followed by agarose gel electrophoresis (Maniatis *ibid*, Example 1).

For the displacement assay 300nM streptavidin was added to quench any displaced S-HRP and the presence of S-HRP on the DNA was monitored by ethanol precipitation of the DNA followed by spotting onto nylon membrane and assaying for chemiluminescence as above.

All *Xho*I digestions produced cleavage of the linear DNA into fragments of the predicted sizes, indicating that translocation did not 'bury' the *Xho*I site. This suggests that the previous results were a consequence of using circular plasmid DNA.

The displacement assays showed a lack of, or significantly reduced amounts of, chemiluminescence indicating loss of HRP from the DNA. Controls of no ATP added, no *Eco*R124I (motor) and use of γ-S ATP (a non-hydrolysable analogue of ATP that prevents translocation) indicated that the excess streptavidin did not displace the bound streptavidin-HRP except when translocation occurred.

### Example 5B - Lack of Displacement of Biotin-Streptavidin when Bound towards end of Linear DNA

The above experiments were repeated but with the biotin attached to the very end of a linear DNA. A modified version of the oligoduplex described in Example 4A was employed for these experiments. The first few bases of each upper strand were altered to produce a PstI "sticky end".

The experiments were as described in Example 2 except that the oligoduplex was ligated to the PstI site of pCFD30 DNA and, to ensure the correct arrangement of the s_{R124} recognition site and biotin, the plasmid was further cleaved with SalI. Purification of the large fragment using standard techniques of phenol extraction of low melting point agarose gels resulted in a linear DNA molecule with biotin attached to one end and the s_{R124} site at the other end. In this case no displacement of the HRP was observed under any of the conditions described above. Therefore, when the biotin is "free" at the very end of a DNA molecule it is not displaced by the translocating complex.

### Example 5 - Conclusions

The observation in Example 2 that the *Xho*I site, present on the circular plasmid DNA, was 'buried' by the stalled translocating complex was not due to the presence of the biotin-S-HRP blocking translocation, but to the stalling of the complex following translocation of all available DNA. Nevertheless, Example 2 still demonstrates that translocation, by the R₁-complex, occurs.

Therefore, while we have confirmed that the R₁-complex is capable of translocation, unexpectedly it is also able to displace the very strong biotin-streptavidin linkage. However, when the biotin is attached to any of the terminal few nucleotides of the DNA, the biotin-streptavidin linkage is not disrupted.

The *Eco*R124 R₁-complex used was produced using excess Stp to ensure little or no R₂-complex was formed. However, the same results were obtained when, in place of HsdR(R124), HsdR(prr) in the absence of Stp was used. Example 3 shows that the R₂-complex is bi-directional; all motors were displaced from the chip surface. It has also been found ( Firman et al in Eur Mol Biol Org J 19(9) 2094-2102 (2000)) that the R₁-complex is less processive (resulting in a slower translocation rate) than the R₂-complex.

### Example 6 - Molecular Dynamo

The plasmid used in example 2A was used to attach a streptavidin- coated paramagnetic bead (available from Pierce & Warriner). DNA carrying the attached bead was purified from solution using a magnet following the manufacturer's instructions. This DNA, linearised by cleavage with PstI and with the attached paramagnetic bead was used to determine whether the molecular motor would rotate the paramagnetic bead in solution.

The DNA and motor were prepared as described in Example 1 and the sample loaded into the capillary tube of a paramagnetic resonance machine. 2mM ATP was added to one of the tubes and allowed to diffuse into the sample. As the ATP entered the measuring cell, a paramagnetic moment was measured, which gradually weakened as the ATP diffused through the sample. Addition of χ-S-ATP produced no such signal, indicating that ATP hydrolysis is required for this effect.

The bead "spinning" within the applied magnetic field produces a paramagnetic moment. This reflects translocation of the DNA (the free end of the DNA rotates as the motor follows the double helix) and could be used to measure translocation. Furthermore, it indicates the possibility of replacing the magnetic bead with a permanent magnetic bead and using this system as a molecule dynamo - if the DNA is surface-attached the spinning magnetic bead should generate electricity within the DNA "conductor".

It will be apparent to a person skilled in the art that the above-described system has applications in the screening or testing for a pre-determined biological, chemical or physical activity; for example, in screening for new pharmacologically-effective ligands.

## Claims

1. A molecular motor system comprising a nucleic acid sequence having bound thereto:
(1) at a first, proximal, region of the nucleic acid, an enzyme capable of translocating the nucleic acid sequence, said enzyme remaining bound to said proximal region of the nucleic acid, as a complex therewith, during the translocation; and
(2) at a second, distal, region of the nucleic acid, a bound substance capable of remaining bound to the nucleic acid sequence at said distal region during the translocation;
**characterised in that** the enzyme comprises a type I restriction-modification enzyme having HsdR, HsdS and HsdM sub-units exhibiting the stoichiometric form HsdR₁M₂S₁.

2. A system according to Claim 1, wherein the nucleic acid sequence comprises a circular or linear DNA sequence.

3. A system according to Claim 1, wherein the bound substance comprises a binding ligand that can bind to a substance in solution.

4. A system according to Claim 1, wherein the nucleic acid is attached to a solid support.

5. A system according to Claim 3, wherein the nucleic acid is linear, thus having two ends, the bound substance being bound at one end and a solid support being bound at the other end.

6. A system according to Claim 3, wherein the bound substance comprises one or more of:
(a) a substance which is required to be translocated; or
(b) means for binding to the nucleic acid-enzyme complex the substance which is required to be translocated; or
(c) both (a) and (b) together.

7. A system according to Claim 3, wherein the bound substance comprises one or more of:
(a) a binding ligand for binding a substance in solution, suspension or dispersion;
(b) an enzyme which produces chemiluminescence;
(c) a magnetic substance;
(d) a DNA sequence;
(e) a scintillant;
(f) a radioactive substance;
(g) a substance capable of producing an electric current;
(h) a substance capable of movement or resulting in movement;
(i) a substance capable of interacting with the environment of the system to produce a detectable and/or measurable effect; and/or
(j) biotin, streptavidin or avidin.

8. A molecular motor system comprising a nucleic acid sequence having bound thereto:
(1) an enzyme capable of translocating the nucleic acid sequence, said enzyme remaining bound to a proximal region of the nucleic acid, as a complex therewith, during the translocation, and
(2) a solid support;
**characterised in that** the enzyme comprises a type I restriction-modification enzyme having HsdR, HsdS and HsdM sub-units exhibiting the stoichiometric form HsdR₁M₂S₁.

9. A system according to claim 1 or claim 8, wherein the HsdR sub-unit is that of the type IC restriction-modification enzyme EcoprrI or a mutant of said HsdR sub-unit which imparts to the enzyme the property of translocating a nucleic acid sequence without causing cleavage thereof, and without loss of ATPase activity.

10. A method for translocating a substance bound to a nucleic acid sequence from a distal region of the nucleic acid sequence towards a proximal region, which method comprises:
(a) (i) providing at the distal region of the nucleic acid sequence a bound substance, or
(ii) binding a substance to the distal region of the nucleic acid sequence; and
(b) (i) providing at the proximal region a complex of the nucleic acid sequence with an enzyme, or
(ii) complexing an enzyme to the proximal region of the nucleic acid sequence, and
(c) activating the enzyme, whereby the enzyme translocates the nucleic acid sequence, including the bound substance, from the distal region towards the proximal region;
**characterised in that** the enzyme comprises a type I restriction-modification enzyme having HsdR, HsdS and HsdM sub-units exhibiting the stoichiometric form HsdR₁M₂S₁.

11. A method according to Claim 10, wherein step (c) is carried out in the presence of ATP and Mg⁺⁺ ions.

12. A method according to Claim 10, wherein step (c) is carried out in the presence of a restriction buffer.

13. A method according to Claim 10, wherein step (c) is carried out in the presence of dithiothreitol.

14. A method according to Claim 10, wherein the polynucleotide-enzyme complex is attached to a solid support.

15. A method according to Claim 10, wherein the nucleic acid sequence comprises a circular or linear DNA sequence.

16. A method according to Claim 15, wherein the HsdR sub-unit is that of the type IC restriction-modification enzyme *Eco*prrI or a mutant of said HsdR sub-unit which stabilises the R₁ complex.

17. A method for capturing a test substance in solution, suspension or dispersion and bringing it into association with a solid surface, which method comprises:
(a) providing a molecular motor system comprising a nucleic acid having:
(i) a proximal region of the nucleic acid to which is bound an enzyme capable of translocating the nucleic acid sequence, said enzyme remaining bound to said proximal region of the nucleic acid, as a complex therewith, during the translocation;
(ii) a distal region of the nucleic acid which enables the test substance to be captured, the distal region and/or the test substance being adapted for that purpose, and
(iii) a solid surface attached to the nucleic acid;
(b) bringing the distal region of the polynucleotide-enzyme complex into contact with the test substance, whereby the test substance is captured, and
(c) activating the enzyme, whereby the enzyme translocates the polynucleotide, including the test substance, from the distal region towards the solid surface;
**characterised in that** the enzyme comprises a type I restriction-modification enzyme having HsdR, HsdS and HsdM sub-units exhibiting the stoichiometric form HsdR₁M₂S₁.

18. A method for screening a test substance for a predetermined biological, chemical or physical activity, which method comprises:
(a) providing a solution, suspension or dispersion of the test substance, either (i) itself or (ii) in association with a first interactive substance, capable of providing or inducing a detectable reaction in a second interactive substance;
(b) providing a polynucleotide motor system comprising a nucleic acid having bound thereto:
(1) at a first, proximal region of the nucleic acid, an enzyme capable of translocating the nucleic acid sequence, said enzyme remaining bound to said proximal region of the nucleic acid, as a complex therewith, during the translocation, and
(2) at a second, distal region of the nucleic acid, a bound substance capable of remaining bound to the nucleic acid sequence at said distal region during the translocation;
wherein the nucleic acid is attached to a solid support and wherein:
(i) the bound substance is further capable of binding to a test substance exhibiting the predetermined activity, and
(ii) the bound substance is itself or the solid support comprises the second interactive substance;
(c) activating the polynucleotide motor system to effect translocation, and
(d) monitoring the presence or absence of the detectable reaction during or after translocation;
**characterised in that** the enzyme comprises a type I restriction-modification enzyme having HsdR, HsdS and HsdM sub-units exhibiting the stoichiometric form HsdR₁M₂S₁.

19. Use of:
a type I restriction-modification enzyme having HsdR, HsdM and HsdS sub-units in the stoichiometric form HsdR₁M₂S₁
in a molecular motor system as claimed in any one of claims 1 to 9.

20. Use of an enzyme according to Claim 19 wherein the enzyme is bound to a nucleic acid sequence.

21. Use of an enzyme according to Claim 20 wherein the nucleic acid sequence comprises a circular or linear DNA sequence.

22. Use of an enzyme according to any one of claims 19 to 21, wherein the HsdR sub-unit is of the type IC restriction-modification enzyme *Eco*prrI or is a mutant of said *EcoprrI* HsdR sub-unit that stabilises the R₁ complex.

## Patentansprüche

1. Molekulares Motorsystem, umfassend eine Nucleinsäuresequenz, die
(1) an einer ersten, proximalen Region der Nucleinsäure ein daran gebundenes Enzym aufweist, das die Nucleinsäuresequenz translozieren kann, wobei das Enzym während der Translokation an der proximalen Region der Nucleinsäure in Form eines Komplexes mit derselben gebunden bleibt, und
(2) an einer zweiten, distalen Region der Nucleinsäure eine daran gebundene Substanz aufweist, die imstande ist, während der Translokation an der Nucleinsäuresequenz an der distalen Region gebunden zu bleiben,
**dadurch gekennzeichnet, dass** das Enzym ein Restriktions-Modifikationsenzym vom Typ I mit HsdR-, HsdS- und HsdM-Untereinheiten umfasst, die die stöchiometrische Form HsdR₁M₂S₁ aufweisen.

2. System nach Anspruch 1, wobei die Nucleinsäuresequenz eine ringförmige oder lineare DNA-Sequenz umfasst.

3. System nach Anspruch 1, wobei die gebundene Substanz einen bindenden Liganden umfasst, der an eine Substanz in Lösung binden kann.

4. System nach Anspruch 1, wobei die Nucleinsäure an einen festen Träger gebunden ist.

5. System nach Anspruch 3, wobei die Nucleinsäure linear ist und somit zwei Enden aufweist, wobei die gebundene Substanz an einem Ende gebunden ist und der feste Träger am anderen Ende gebunden ist.

6. System nach Anspruch 3, wobei die gebundene Substanz folgendes umfasst:
(a) eine oder mehrere Substanzen, die zu translozieren sind; oder
(b) ein oder mehrere Hilfsmittel zum Binden der zu translozierenden Substanz an den Nucleinsäure-Enzym-Komplex, oder
(c) sowohl (a) als auch (b) zusammen.

7. System nach Anspruch 3, wobei die gebundene Substanz folgendes umfasst:
(a) einen oder mehrere bindende Liganden zur Bindung einer Substanz in Lösung, Suspension oder Dispersion;
(b) ein oder mehrere Enzyme, die Chemilumineszenz erzeugen;
(c) eine oder mehrere magnetische Substanzen;
(d) eine oder mehrere DNA-Sequenzen;
(e) eine oder mehrere Szintillationssubstanzen;
(f) eine oder mehrere radioaktive Substanzen;
(g) eine oder mehrere Substanzen, die einen elektrischen Strom erzeugen können;
(h) eine oder mehrere Substanzen, die bewegungsfähig sind oder zu einer Bewegung führen;
(i) eine oder mehrere Substanzen, die mit der Umgebung des Systems wechselwirken, um eine nachweisbare und/oder messbare Wirkung zu ergeben; und/oder
(j) eines oder mehrere aus Biotin, Streptavidin oder Avidin.

8. Molekulares Motorsystem, umfassend eine Nucleinsäuresequenz, die folgendes daran gebunden aufweist:
(1) ein Enzym, das die Nucleinsäuresequenz translozieren kann, wobei das Enzym während der Translokation an der proximalen Region der Nucleinsäure in Form eines Komplexes mit derselben gebunden bleibt, und
(2) einen festen Träger;
**dadurch gekennzeichnet, dass** das Enzym ein Restriktions-Modifikationsenzym vom Typ I mit HsdR-, HsdS- und HsdM-Untereinheiten umfasst, die die stöchiometrische Form HsdR₁M₂S₁ aufweisen.

9. System nach Anspruch 1 oder 8, wobei die HsdR-Untereinheit diejenige des Typ IC-Restriktions-Modifikationsenzyms EcoprrI oder eine Mutante der HsdR-Untereinheit ist, die dem Enzym die Eigenschaft verleiht, eine Nucleinsäuresequenz ohne Herbeiführung einer Spaltung derselben und ohne Verlust der ATPase-Aktivität zu translozieren.

10. Verfahren zur Translokation einer an eine Nucleinsäuresequenz gebundenen Substanz von einer distalen Region der Nucleinsäuresequenz zu einer proximalen Region, umfassend:
(a) (i) Bereitstellen einer gebundenen Substanz an der distalen Region der Nucleinsäuresequenz, oder
(ii) Binden einer Substanz an die distale Region der Nucleinsäuresequenz, und
(b) (i) Bereitstellen eines Komplexes der Nucleinsäuresequenz mit einem Enzym an der proximalen Region, oder
(ii) Komplexieren eines Enzyms an die proximale Region der Nucleinsäuresequenz, und
(c) Aktivieren des Enzyms, wodurch das Enzym die Nucleinsäuresequenz einschließlich der gebundenen Substanz von der distalen Region zur proximalen Region transloziert;
**dadurch gekennzeichnet, dass** das Enzym ein Restriktions-Modifikationsenzym vom Typ I mit HsdR-, HsdS- und HsdM-Untereinheiten umfasst, die die stöchiometrische Form HsdR₁M₂S₁ aufweisen.

11. Verfahren nach Anspruch 10, wobei der Schritt (c) in Gegenwart von ATP und Mg²⁺-Ionen durchgeführt wird.

12. Verfahren nach Anspruch 10, wobei der Schritt (c) in Gegenwart eines Restriktionspuffers durchgeführt wird.

13. Verfahren nach Anspruch 10, wobei der Schritt (c) in Gegenwart von Dithiothreitol durchgeführt wird.

14. Verfahren nach Anspruch 10, wobei der Polynucleotid-Enzym-Komplex an einen festen Träger gebunden ist.

15. Verfahren nach Anspruch 10, wobei die Nucleinsäuresequenz eine ringförmige oder lineare DNA-Sequenz umfasst.

16. Verfahren nach Anspruch 15, wobei die HsdR-Untereinheit diejenige des Typ IC-Restriktions-Modifikationsenzyms EcoprrI oder eine Mutante der HsdR-Untereinheit ist, die den R₁-Komplex stabilisiert.

17. Verfahren zum Einfang einer Testsubstanz in Lösung, Suspension oder Dispersion und Verbinden derselben mit einer festen Oberfläche, umfassend:
(a) Bereitstellen eines molekularen Motorsystems, umfassend eine Nucleinsäure mit
(i) einer proximalen Region der Nucleinsäure, an die ein Enzym gebunden ist, das die Nucleinsäuresequenz translozieren kann,
wobei das Enzym während der Translokation an der proximalen Region der Nucleinsäure in Form eines Komplexes mit derselben gebunden bleibt,
(ii) einer distalen Region der Nucleinsäure, die das Einfangen der Testsubstanz ermöglicht, wobei die distale Region und/oder die Testsubstanz für diesen Zweck angepasst sind, und
(iii) einer festen Oberfläche, die an die Nucleinsäure gebunden ist,
(b) Inkontaktbringen der distalen Region des Polynucleotid-Enzym-Komplexes mit der Testsubstanz, wodurch die Testsubstanz eingefangen wird, und
(c) Aktivieren des Enzyms, so dass das Enzym das Polynucleotid einschließlich der Testsubstanz von der distalen Region zur festen Oberfläche transloziert;
**dadurch gekennzeichnet, dass** das Enzym ein Restriktions-Modifikationsenzym vom Typ I mit HsdR-, HsdS- und HsdM-Untereinheiten umfasst, die die stöchiometrische Form HsdR₁M₂S₁ aufweisen.

18. Verfahren zum Screening einer Testsubstanz auf eine vorbestimmte biologische, chemische oder physikalische Aktivität, umfassend:
(a) Bereitstellen einer Lösung, Suspension oder Dispersion der Testsubstanz (i) selbst oder (ii) in Verbindung mit einer ersten wechselwirkenden Substanz, die in einer zweiten wechselwirkenden Substanz eine nachweisbare Reaktion ergeben oder induzieren kann,
(b) Bereitstellen eines Polynucleotid-Motorsystems, umfassend eine Nucleinsäure, die
(1) an einer ersten, proximalen Region der Nucleinsäure ein daran gebundenes Enzym aufweist, das die Nucleinsäuresequenz translozieren kann, wobei das Enzym während der Translokation an der proximalen Region der Nucleinsäure in Form eines Komplexes mit derselben gebunden bleibt, und
(2) an einer zweiten, distalen Region der Nucleinsäure eine daran gebundene Substanz aufweist, die imstande ist, während der Translokation an der Nucleinsäuresequenz an der distalen Region gebunden zu bleiben,
wobei die Nucleinsäure an einen festen Träger gebunden ist und wobei:
(i) die gebundene Substanz des Weiteren an eine Testsubstanz binden kann, welche die vorbestimmte Aktivität aufweist, und
(ii) die gebundene Substanz selbst die zweite wechselwirkende Substanz ist oder der feste Träger diese umfasst;
(c) Aktivieren des Polynucleotid-Motorsystems, um die Translokation herbeizuführen, und
(d) Überwachen des Vorhandenseins oder Fehlens der nachweisbaren Reaktion während oder nach der Translokation;
**dadurch gekennzeichnet, dass** das Enzym ein Restriktions-Modifikationsenzym vom Typ I mit HsdR-, HsdS- und HsdM-Untereinheiten umfasst, die die stöchiometrische Form HsdR₁M₂S₁ aufweisen.

19. Verwendung eines Restriktions-Modifikationsenzyms vom Typ I mit HsdR-, HsdS- und HsdM-Untereinheiten in der stöchiometrischen Form HsdR₁M₂S₁ in einem molekularen Motorsystem nach einem der Ansprüche 1 bis 9.

20. Verwendung eines Enzyms nach Anspruch 19, wobei das Enzym an eine Nucleinsäuresequenz gebunden ist.

21. Verwendung eines Enzyms nach Anspruch 20, wobei die Nucleinsäuresequenz eine ringförmige oder lineare DNA-Sequenz umfasst.

22. Verwendung eines Enzyms nach einem der Ansprüche 19 bis 21, wobei die HsdR-Untereinheit diejenige des Typ IC-Restriktions-Modifikationsenzyms *Eco*prrI oder eine Mutante der *Eco*prrI-HsdR-Untereinheit ist, die den R₁-Komplex stabilisiert.

## Revendications

1. Système de moteur moléculaire comprenant une séquence d'acide nucléique à laquelle sont liés :
(1) à une première région proximale de l'acide nucléique, une enzyme apte à la translocation de la séquence d'acide nucléique, ladite enzyme restant liée à ladite région proximale de l'acide nucléique, sous forme d'un complexe avec celui-ci, au cours de la translocation ; et
(2) à une seconde région distale de l'acide nucléique, une substance clé capable de rester liée à la séquence d'acide nucléique à ladite région distale au cours de la translocation ;
**caractérisé en ce que** l'enzyme comprend une enzyme de modification de restriction de type I comprenant des sous-unités HsdR, HsdS et HsdM sous la forme stoechiométrique HsdR₁M₂S₁.

2. Système suivant la revendication 1, dans lequel la séquence d'acide nucléique comprend une séquence d'ADN circulaire ou linéaire.

3. Système suivant la revendication 1, dans lequel la substance liée comprend un ligand de liaison qui peut se lier à une substance en solution.

4. Système suivant la revendication 1, dans lequel l'acide nucléique est fixé à un support solide.

5. Système suivant la revendication 3, dans lequel l'acide nucléique est linéaire, ce qui fait qu'il possède deux extrémités, la substance liée étant liée à une extrémité et un support solide étant lié à l'autre extrémité.

6. Système suivant la revendication 3, dans lequel la substance liée comprend une ou plusieurs des entités consistant en :
(a) une substance qui est nécessaire pour subir la translocation ; ou
(b) un moyen pour la liaison au complexe acide nucléique-enzyme de la substance qui est nécessaire pour subir la translocation ; ou
(c) (a) et (b) ensemble.

7. Système suivant la revendication 3, dans lequel la substance liée comprend une ou plusieurs des entités consistant en :
(a) un ligand de liaison pour la liaison d'une substance en solution, suspension ou dispersion ;
(b) une enzyme qui produit de la chimioluminescence ;
(c) une substance magnétique ;
(d) une séquence d'ADN ;
(e) un agent de scintillation ;
(f) une substance radioactive ;
(g) une substance capable de produire un courant électrique ;
(h) une substance capable de présenter un mouvement ou ayant pour résultat un mouvement ;
(i) une substance capable d'interagir avec l'environnement du système pour produire un effet détectable et/ou mesurable ; et/ou
(j) de la biotine, de la streptavidine ou de l'avidine.

8. Système de moteur moléculaire comprenant une séquence d'acide nucléique à laquelle sont liés :
(1) une enzyme apte à la translocation de la séquence d'acide nucléique, ladite enzyme restant liée à une région proximale de l'acide nucléique, sous forme d'un complexe avec celui-ci, au cours de la translocation, et
(2) un support solide ;
**caractérisé en ce que** l'enzyme comprend une enzyme de modification de restriction de type I comprenant des sous-unités HsdR, HsdS et HsdM sous la forme stoechiométrique HsdR₁M₂S₁.

9. Système suivant la revendication 1 ou la revendication 8, dans lequel la sous-unité HsdR est celle de l'enzyme de modification de restriction de type IC EcoprrI ou un mutant de ladite sous-unité HsdR qui confère à l'enzyme la propriété de translocation d'une séquence d'acide nucléique sans provoquer son clivage, et sans perte d'activité d'ATPase.

10. Procédé pour la translocation d'une substance liée à une séquence d'acide nucléique d'une région distale de la séquence d'acide nucléique vers une région proximale, procédé qui comprend les étapes consistant à :
(a) (i) fournir à la région distale de la séquence d'acide nucléique une substance liée, ou
(ii) lier une substance à la région distale de la séquence d'acide nucléique ; et
(b) (i) fournir à la région proximale un complexe de la séquence d'acide nucléique avec une enzyme, ou
(ii) complexer une enzyme à la région proximale de la séquence d'acide nucléique, et
(c) activer l'enzyme, l'enzyme provoquant ainsi la translocation de la séquence d'acide nucléique, y compris de la substance liée, de la région distale vers la région proximale ;
**caractérisé en ce que** l'enzyme comprend une enzyme de modification de restriction de type I comprenant des sous-unités HsdR, HsdS et HsdM sous la forme stoechiométrique HsdR₁M₂S₁.

11. Procédé suivant la revendication 10, dans lequel l'étape (c) est mise en oeuvre en présence d'ATP et d'ions Mg⁺⁺.

12. Procédé suivant la revendication 10, dans lequel l'étape (c) est mise en oeuvre en présence d'un tampon de restriction.

13. Procédé suivant la revendication 10, dans lequel l'étape (c) est mise en oeuvre en présence de dithiothréitol.

14. Procédé suivant la revendication 10, dans lequel le complexe polynucléotide-enzyme est fixé à un support solide.

15. Procédé suivant la revendication 10, dans lequel la séquence d'acide nucléique comprend une séquence d'ADN circulaire ou linéaire.

16. Procédé suivant la revendication 15, dans lequel la sous-unité HsdR est celle de l'enzyme de modification de restriction de type IC EcoprrI ou un mutant de ladite sous-unité HsdR qui stabilise le complexe R₁.

17. Procédé pour capturer une substance d'essai en solution, suspension ou dispersion et la mettre en association avec une surface solide, procédé comprenant les étapes consistant à :
(a) fournir un système de moteur moléculaire comprenant un acide nucléique ayant :
(i) une région proximale de l'acide nucléique à laquelle est liée une enzyme capable d'effectuer la translocation de la séquence d'acide nucléique, ladite enzyme restant liée à ladite région proximale de l'acide nucléique, sous forme d'un complexe avec celui-ci, au cours de la translocation ;
(ii) une région distale de l'acide nucléique qui permet à la substance d'essai d'être capturée, la région distale et/ou la substance d'essai étant adaptée à cette fin, et
(iii) une surface solide fixée à l'acide nucléique ;
(b) mettre la région distale du complexe polynucléotide-enzyme en contact avec la substance d'essai, la substance d'essai étant ainsi capturée, et
(c) activer l'enzyme, l'enzyme provoquant ainsi la translocation du polynucléotide, y compris de la substance d'essai, de la région distale vers la surface solide ;
**caractérisé en ce que** l'enzyme comprend une enzyme de modification de restriction de type I comprenant des sous-unités HsdR, HsdS et HsdM sous la forme stoechiométrique HsdR₁M₂S₁.

18. Procédé pour sélectionner une substance d'essai pour une activité biologique, chimique ou physique prédéterminée, procédé qui comprend les étapes consistant à :
(a) fournir une solution, suspension ou dispersion de la substance d'essai, (i) telle quelle ou (ii) en association avec une première substance interactive, capable d'engendrer ou d'induire une réaction détectable dans une seconde substance interactive ;
(b) fournir un système de moteur polynucléotidique comprenant un acide nucléique auquel sont liés :
(1) à une première région, proximale, de l'acide nucléique, une enzyme apte à la translocation de la séquence d'acide nucléique, ladite enzyme restant liée à ladite région proximale de l'acide nucléique, sous forme d'un complexe avec celui-ci, au cours de la translocation ; et
(2) à une seconde région, distale, de l'acide nucléique, une substance clé capable de rester liée à la séquence d'acide nucléique à ladite région distale au cours de la translocation ;
dans lequel l'acide nucléique est fixé à un support solide et dans lequel :
(i) la substance liée est en outre capable de se lier à une substance d'essai présentant l'activité prédéterminée, et
(ii) la substance liée est présente telle quelle ou bien le support solide comprend la seconde substance interactive ;
(c) activer le système de moteur polynucléotidique pour effectuer la translocation, et
(d) contrôler la présence ou l'absence de la réaction détectable pendant ou après la translocation ;
**caractérisé en ce que** l'enzyme comprend une enzyme de modification de restriction de type I comprenant des sous-unités HsdR, HsdS et HsdM sous la forme stoechiométrique HsdR₁M₂S₁.

19. Utilisation :
d'une enzyme de modification de restriction de type I comprenant des sous-unités HsdR, HsdS et HsdM sous la forme stoechiométrique HsdR₁M₂S₁,
dans un système de moteur moléculaire suivant l'une quelconque des revendications 1 à 9.

20. Utilisation d'une enzyme suivant la revendication 19, dans laquelle l'enzyme est liée à une séquence d'acide nucléique.

21. Utilisation d'une enzyme suivant la revendication 20, dans laquelle la séquence d'acide nucléique comprend une séquence d'ADN circulaire ou linéaire.

22. Utilisation d'une enzyme suivant l'une quelconque des revendications 19 à 21, dans laquelle la sous-unité HsdR est une sous-unité de l'enzyme de modification de restriction de type IC *Eco*prrI ou est un mutant de ladite sous-unité HdsR de *Eco*prrI qui stabilise le complexe R₁.
